# EUROPEAN PATENT APPLICATION

(11) **EP 4 238 555 A2**
(43) Date of publication of application: **06.09.2023**
(21) Application number: 23177087.6
(22) Date of filing: 29.05.2020
(51) Int. Cl.: A61K 9/00, A61K 9/20, A61K 31/13, A61K 31/445, A61P 25/28

(54) **IMMEDIATE RELEASE FIXED-DOSE COMBINATION OF MEMANTINE AND DONEPEZIL**

(30) Priority: 31.05.2019 PT 2019115557
(62) Divisional of application: 20732664.6
(71) Applicant: Tecnimede, Sociedade Técnico-Medicinal, SA, 2710-089 Sintra (PT)
(72) Inventor: OLIVEIRA MACHUCO ESTEVENS, MARIA CATARINA, 2710-089 SINTRA (PT); SILVA MARQUES DA COSTA, RICARDO MANUEL, 2710-089 SINTRA (PT); SILVA SERRA, JOÃO PEDRO, 2710-089 SINTRA (PT); PARDAL FILIPE, AUGUSTO EUGÉNIO, 1800-331 LISBOA (PT)
(74) Representative: Patentree

(57) **Abstract**

The present disclosure relates immediate release fixed-dosed combination pharmaceutical composition comprising the active pharmaceutical ingredients Memantine and Donepezil for the treatment of moderate to severe Alzheimer's disease.

## Description

### TECHNICAL FIELD

The present disclosure relates to an immediate release fixed-dose combination pharmaceutical composition comprising Donepezil and Memantine, for the treatment of Alzheimer's disease.

### BACKGROUND

According to the United States Pharmacopoeia (USP), immediate release solid oral formulations containing Donepezil hydrochloride or Memantine hydrochloride refers to formulations which exhibit a drug dissolution of not less than 80% within 30 minutes in 0.1 N hydrochloric acid.

Donepezil is the international non-proprietary name (INN) for (RS)-2-[(1-benzyl-4-piperidyl)methyl]-5,6-dimethoxy-2,3-dihydroinden-1-one which has the following chemical structure:

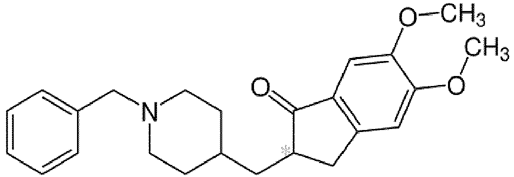

Memantine is the INN for 3,5-dimethyladamantan-1-amine which has the following chemical structure:

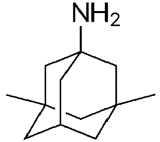

Donepezil is an acetylcholinesterase inhibitor. Pharmaceutical compositions of Donepezil in its hydrochloride salt form are registered and marketed under the trade name ARICEPT^{®} for the treatment of mild to moderately severe Alzheimer's dementia.

Memantine is a N-Methyl-D-Aspartate (NMDA) receptor antagonist. Pharmaceutical compositions of Memantine in its hydrochloride salt form are registered and marketed under the trade name EBIXA^{®} for the treatment of moderate to severe Alzheimer's disease.

The document WO 03/101458 discloses pharmaceutical compositions comprising acetylcholinesterase inhibitors and N-Methyl-D-Aspartate (NMDA) receptor antagonists. However, no specific pharmaceutical formulation of Donepezil or Memantine is disclosed. Further, no fixed-dose composition was disclosed.

The document WO 2004/037234 discloses combination therapy using 1-aminocyclohexane derivatives such as Memantine or Neramexane, together with acetylcholinesterase inhibitors (AChEI) such as Galantamine, Tacrine, Donepezil, or Rivastigmine, for treating dementia by inducing cognitive improvements. No specific pharmaceutical formulations of Donepezil or Memantine, or any fixed dosed composition are disclosed. From the examples it is clear that the active ingredients are provided in separate units.

The document WO 2006/121560 discloses fixed-dose pharmaceutical compositions of Donepezil and Memantine wherein one or both agents are provided in an extended release dosage form, i.e. with an *in vitro* dissolution profile of less than 70% in one hour, using USP type 2 (paddle) dissolution system at 50 rpm, at a temperature of 37 °C with water as the dissolution medium.

There is no guidance in the prior art on how to design, develop and manufacture oral fixed-dose pharmaceutical compositions containing immediate release Memantine and Donepezil, for the treatment or therapy of moderate to severe Alzheimer's disease. In particular, there is no guidance on how to design, develop and manufacture said pharmaceutical composition such that it simultaneously exhibits a plurality of characteristics that might render the same compositions amenable for commercial scale manufacturing.

There are several obstacles which the inventors need to overcome during the development of a fixed-dose combination product comprising the active ingredients Donepezil and Memantine. The inventors of the current application faced unconventional challenges requiring a substantial amount of research activities in order to develop a fixed-dose combination pharmaceutical composition amenable for commercial scale manufacturing. Specifically, a composition which concurrently exhibits suitable stability, cost effectiveness, and process robustness (good uniformity, good flowability, good compact ability/compressibility, mass uniformity, and adequate hardness). At the same time, the novel fixed-dose combination composition must exhibit pharmacokinetics comparable to products containing only either Donepezil or Memantine that is currently available in the market.

The current Donepezil (ARICEPT^{®}) and Memantine (EBIXA^{®}) compositions on the market have different formulations and utilize different excipients. This shows that it is possible to manufacture compositions of Donepezil and Memantine using different techniques.

In the design of fixed-dose combination pharmaceutical compositions, straightforward approaches such as merely combining the excipients in an additive manner, are undeniably rare or even non-existent. Should the skilled person be tasked to develop a fixed-dose combination composition that is bioequivalent to mono-products, the obvious method would be to include, as far as possible, all the ingredients present in both mono-products. However, the abovementioned method lacks any practical applicability as the excipients chosen must have very clear function. That is, particular excipient should be chosen for particular specific effect. The presence of a particular excipient should be demonstrated to exert the required effect. The commercially available mono-products, their composition and their excipients are listed in table 1 according to their respective Summary of Product Characteristics (SmPC).

Table 1 - commercially available mono-products, their composition and their excipients are listed in table 1 according to their respective Summary of Product Characteristics.

| Axura^{®} | Aricept^{®} | Function |
|---|---|---|
| Memantine hydrochloride | Donepezil hydrochloride | Drug substance |
| - | Lactose monohydrate | Diluent / filler |
| Microcrystalline cellulose | Microcrystalline cellulose | Diluent / filler |
| Croscarmellose sodium | - | Disintegrant |
| - | Maize starch | Disintegrant |
| Silica colloidal, anhydrous | - | Glidant |
| - | Hydroxypropylcellulose | Binder |
| Magnesium stearate | Magnesium stearate | Lubricant |

Analyzing the compositions described for the commercial mono-products, it becomes evident that merely adding the compositions together is not a rational or practical approach for developing a fixed-dose combination composition of the compositions.

The skilled person is aware of the suitability of a particular excipient for a specific manufacturing process. In addition, taking into account the very poor flow and compaction properties of Memantine and Donepezil, the immediate approach will be to process the powders by means of a high-shear granulation, followed by the required downstream steps to convert the granulated material into a solid oral dosage form. Once a wet granulation process is deemed required, a person skilled in the art will use a plurality of excipients to enhance the processability. The combination of lactose monohydrate with microcrystalline cellulose in solid-oral dosage forms is common practice. When properly formulated, the fragmenting properties of lactose monohydrate, in combination with the highly plastic behavior of microcrystalline cellulose, yields tablets of high mechanical strength; a mandatory property in order for the tablets to be able to withstand further processing and manipulation (i.e., dedusting, conveying, film coating and or packaging). As a result of the required manufacturing process, the most straightforward approach for developing a fixed-dose combination of Donepezil and Memantine will be to associate the excipients present in Aricept^{®} with both drug compositions.

Memantine is not compatible with some excipients present in ARICEPT^{®}. In particular, Memantine is not compatible with lactose, which is present in the formulation of ARICEPT^{®}. It is known that Memantine and lactose may form a Memantine-Lactose adduct (MLA), through a Maillard reaction (an amine group-containing API, such as Memantine, has been described to react with carbohydrate via a Maillard reaction to afford carbohydrate conjugated adducts). The presence of MLA was confirmed in formulations comprising lactose and Memantine, where substantial amounts of MLA were formed after submitting the formulated product to stress stability studies.

By reducing the amount of lactose in the formulation, it is expected that the amount of MLA is also reduced. The reduction of lactose must be compensated with alternative excipients having similar function/properties as lactose. However, there is no single excipient that is fully similar (biological, chemical and physical properties) to lactose that might replace lactose monohydrate.

Even if one is directed to use polyols (mannitol, xylitol, maltitol, etc.) as an excipient as these excipients possess solubility and compaction properties equivalent to that of lactose, the skilled person is likely to reject the direction. The skill person is one who is aware of two main negative factors that is known in the art.

Firstly, these classes of excipients have detrimental effect on small intestinal transit time, and consequently impacts drug absorption (Guideline for the Investigation of Bioequivalence - CPMP/EWP/QWP/1401/98 Rev. 1/ Corr).

Contrary to all the expectations of one skilled in the art, it is possible to obtain an immediate release oral pharmaceutical composition which satisfy all requirements and is amendable for robust production. Specifically, it is possible to create an immediate release fixed-dose combination composition which when ingested does not produce MLA and has stability and in-vivo bioequivalence that is similar to a composition containing Donepezil or Memantine as sole active ingredient (namely, the current commercial mono-products).

These facts are disclosed in order to illustrate the technical problem addressed by the present disclosure.

### GENERAL DESCRIPTION

The present disclosure relates to an immediate release fixed-dose combination pharmaceutical composition comprising the active pharmaceutical ingredients (APIs) Donepezil and Memantine, for the treatment of moderate to severe Alzheimer's disease.

An aspect of the present disclosure relates to an immediate release oral fixed-dose combination pharmaceutical composition comprising:
from 4.16 mg to 20 mg of Memantine or pharmaceutically acceptable salt; and
from 4.56 mg to 10 mg of Donepezil or pharmaceutically acceptable salt;
wherein Memantine or pharmaceutically acceptable salt and Donepezil or pharmaceutically acceptable salt are the sole active substances.

Another aspect of the present disclosure relates to an immediate release oral fixed-dose combination pharmaceutical composition comprising:
from 4.16 mg to 20 mg of Memantine or pharmaceutically acceptable salt; and
from 4.56 mg to 10 mg of Donepezil or pharmaceutically acceptable salt;
wherein Memantine or pharmaceutically acceptable salt and Donepezil or pharmaceutically acceptable salt are the sole active substances;
wherein the pharmaceutical composition is lactose-free.

Another aspect of the present disclosure is the use of the composition of the present disclosure in treatment or therapy of moderate to severe Alzheimer's disease.

Another aspect of the present disclosure is an immediate release oral fixed-dose combination pharmaceutical composition, for use in treatment or therapy of moderate to severe Alzheimer's disease, comprising:
from 4.16 mg to 20 mg of Memantine or pharmaceutically acceptable salt; and
from 4.56 mg to 10 mg of Donepezil or pharmaceutically acceptable salt;
wherein Memantine or pharmaceutically acceptable salt and Donepezil or pharmaceutically acceptable salt are the sole active substances;
wherein the pharmaceutical composition is lactose-free;
and less than 1.0 wt.% of reducing sugars relative to the total weight of the composition;
wherein the dissolution rate of Memantine and Donepezil in pH 1 dissolution test solution is more than 90% within 5 minutes.

The composition of the present disclosure surprisingly allows an improved dissolution rate, an adequate disintegration rate, and a better shelf storage stability.

In an embodiment for better results, the dissolution rate of Memantine and Donepezil in pH 1 dissolution test solution is more than 91% within 5 minutes; preferably more than 92% within 5 minutes, preferably more than 93% within 5 minutes, preferably more than 94% within 5 minutes; preferably more than 95% within 5 minutes.

The disintegration test is provided to determine whether tablets, capsules or granules disintegrate within the prescribed time when placed in a liquid medium at the experimental conditions. Experimental procedure and the requirements for disintegration differ depending on the pharmaceutical forms groups: uncoated or plain-coated tablets; delayed-release tablets and capsules (ie tablets or capsules that are formulated with acid-resistant or enteric coating); buccal tablets, sublingual tablets, tablets for oral suspension, tablets for oral solution, orally disintegrating tablets(odts) and chewable tablets; effervescent tablets or granules. For orally disintegrating tablets term 'in vitro disintegration time' means that a tablet disintegrates in the oral cavity mainly in a disintegration test with saliva or a disintegration test with a device, usually in 5 to 120 seconds, preferably in 5 to 60 seconds, more preferably in about 5 to 40 seconds, using water at 15-25 °C as the liquid medium. The disintegration test of plain coated or uncoated tablets is performed using water at 37°C, for a maximum time of 15minutes.

In an embodiment for better results, the composition is bioequivalent and the bioequivalence of the composition is given by a 90% confidence interval (CI) for the ratio of geometric means of AUC for plasma donepezil and memantine from 80.00 to 125.00% and, by a 90% CI for the ratio of geometric means of Cmax for plasma donepezil and memantine from 80.00 to 125.00%. Preferably, the bioequivalence of the composition is given by a 90% CI for the ratio of geometric means of AUC for plasma donepezil and memantine from 90.00 to 111.00% and, by a 90% CI for the ratio of geometric means of Cmax for plasma donepezil and memantine from 90.00 to 111.00%.

In an embodiment for better results, the point estimates for the log transformed ratios of AUC and Cmax for plasma donepezil and memantine concentrations are from 95.00% to 105.00%.

In an embodiment for better results, the composition is bioequivalent and the bioequivalence of the composition is given by a 90% CI (Confidence Interval) for the ratio of geometric means of AUC for plasma donepezil and memantine from 80.00 to 125.00% and, by a 90% CI for the ratio of geometric means of Cmax for plasma donepezil and memantine from 80.00 to 125.00%, with the point estimates from 95.00 to 105.00%.

In an embodiment for better results, the composition is bioequivalent and the bioequivalence of the composition is given by a 90% CI for the ratio of geometric means of AUC for plasma donepezil and memantine from 90.00 to 111.00% and, by a 90% CI for the ratio of geometric means of Cmax for plasma donepezil and memantine from 90.00 to 111.00%, with the point estimates from 95.00 to 105.00%.

In an embodiment for better results, the bioequivalence of Memantine in the composition is equivalent to EBIXA^{®}, and the bioequivalence of Donepezil in the composition is equivalent to ARICEPT^{®}.

In an embodiment for better results, the composition of the present disclosure may further comprise mannitol.

In an embodiment for better results, the composition of the present disclosure may comprise from 45.0 wt. % to 70 wt. % of mannitol, preferably from 45.0 wt. % to 60 wt. % of mannitol., more preferably from 45.0 wt. % to 55.0 wt. % of mannitol.

In an embodiment for better results, the composition of the present disclosure may disintegrate in more than or equal to 2 min, using water at 37°C as the liquid medium.

In an embodiment for better results, the pharmaceutically acceptable salt for Memantine is Memantine hydrochloride, preferably in amounts from 5 mg to 20 mg per dose.

In an embodiment for better results, the pharmaceutically acceptable salt for Donepezil is Donepezil hydrochloride, preferably in amounts from 5 mg to 10 mg per dose.

In an embodiment for better results, the composition of the present disclosure may comprise less than 1.0 wt.% of reducing sugars relative to the total weight of the composition, preferably less than 0.6 wt.%, more preferably less than 0.4 wt.%, most preferably less than 0.3 wt.%.

In an embodiment for better results, the dissolution rate of Memantine and Donepezil in pH 1 dissolution test solution is more than 50% within 30 minutes, preferably more than 60% within 30 minutes, most preferably more than 70% within 30 minutes.

In an embodiment for better results, the dissolution rate of Memantine and Donepezil in pH 1 dissolution test solution is more than 92% within 5 minutes, preferably more than 93% within 5 minutes, most preferably more than 95% within 5 minutes.

In an embodiment for better results, the composition of the present disclosure may comprise less than 2.5 wt. % of Memantine-Lactose Adduct (MLA) relative to the weight of Memantine, preferably less than 1.4 wt. %, more preferably less than 1.0 wt. %.

In an embodiment for better results, the composition of the present disclosure may further comprise:
one or more disintegrant(s);
one or more binder(s);
one or more diluents(s);
one or more lubricant(s);
optionally one or more glidant(s);
other pharmaceutically acceptable excipient(s).

In an embodiment for better results, the composition of the present disclosure may comprise
from 2 wt.% to 30 wt.% of one or more disintegrant(s);
from 0.5 wt.% to 10 wt.% of one or more binder(s);
from 5 wt.% to 80 wt.% of one or more diluent(s);
from 0.1 wt.% to 5 wt.% of one or more lubricant(s).

In an embodiment for better results, the composition of the present disclosure may comprise
one or more disintegrant(s) are selected from a list consisting of: starch, carboxymethyl cellulose (carmellose), crosslinked carboxymethyl cellulose (croscarmellose), sodium starch glycolate, low substituted hydroxypropyl cellulose (L-HPC), carboxymethyl starch, polyvinylpyrrolidone, crospovidone, and mixtures thereof;
one or more binders(s) are selected from a list consisting of: hydroxypropyl cellulose (HPC), low-substituted hydroxypropyl cellulose (L-HPC), hydroxypropyl methylcellulose (HPMC) , methyl cellulose, hydroxyethyl cellulose, ethyl cellulose, polyethylene glycol, maltodextrin, pregelatinized starch, polymethacrylates, sodium alginate, polyvinylpyrrolidone (povidone), vinylpyrrolidone/vinylacetate copolymer (copovidone), and mixtures thereof;
one or more diluent(s) are selected from a list consisting of: microcrystalline cellulose (MCC), silicified microcrystalline cellulose (SMCC), sorbitol, lactitol, xylitol, isomalt, dextrin, and mixtures thereof; preferably microcrystalline cellulose;
one or more lubricant(s) are selected from a list consisting of: magnesium stearate, calcium stearate, sodium stearate, stearic acid, sodium glyceryl behenate, sodium stearyl fumarate, and mixtures thereof.

In an embodiment for better results, the composition of the present disclosure may comprise:
from 5 wt. % to 25 wt. % of maize starch;
from 5 wt. % to 20 wt. % of croscarmellose sodium;
from 1 wt. % to 4 wt. % of hydroxypropyl cellulose (HPC);
from 45.0 wt. % to 70 wt. % of mannitol;
from 5 wt. % to 20 wt. % of microcrystalline cellulose;
from 0.1 wt. % to 5 wt. % of magnesium stearate;
wherein all wt. % are relative to the total weight of the composition.

In an embodiment for better results, the composition of the present disclosure is obtainable by wet granulation.

Another aspect of the present disclosure relates to a process for the manufacture of an immediate release oral fixed-dose combination pharmaceutical composition of the present disclosure, comprising the steps of:
preparing a homogenous blend comprising
   Donepezil or a pharmaceutically acceptable salt, in particular Donepezil hydrochloride,
   Memantine or a pharmaceutically acceptable salt, in particular Memantine hydrochloride,
   one or more disintegrant(s), one or more binder(s), and one or more diluents(s);
obtaining granules by means of wet granulation;
drying the wet granules and sieving;
blending the granules with one or more lubricant(s);
compressing the final blend into a tablet;
optionally film-coating the tablet.

### BRIEF DESCRIPTION OF THE DRAWINGS

The following figures provide preferred embodiments for illustrating the disclosure and should not be seen as limiting the scope of invention.

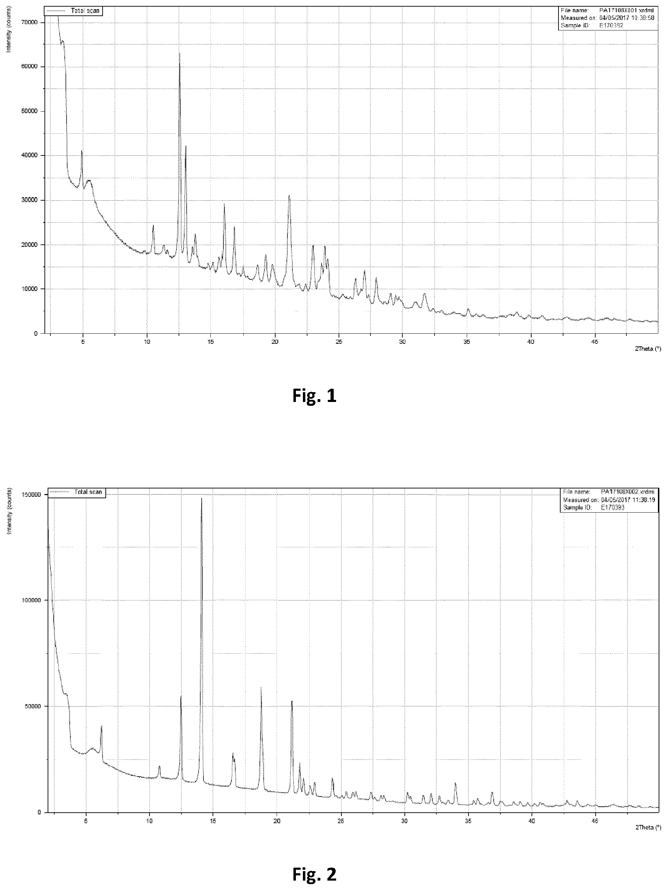

**Figure 1** shows a XRPD of Donepezil hydrochloride form I.
**Figure 2** shows a XRPD of Memantine hydrochloride form I.

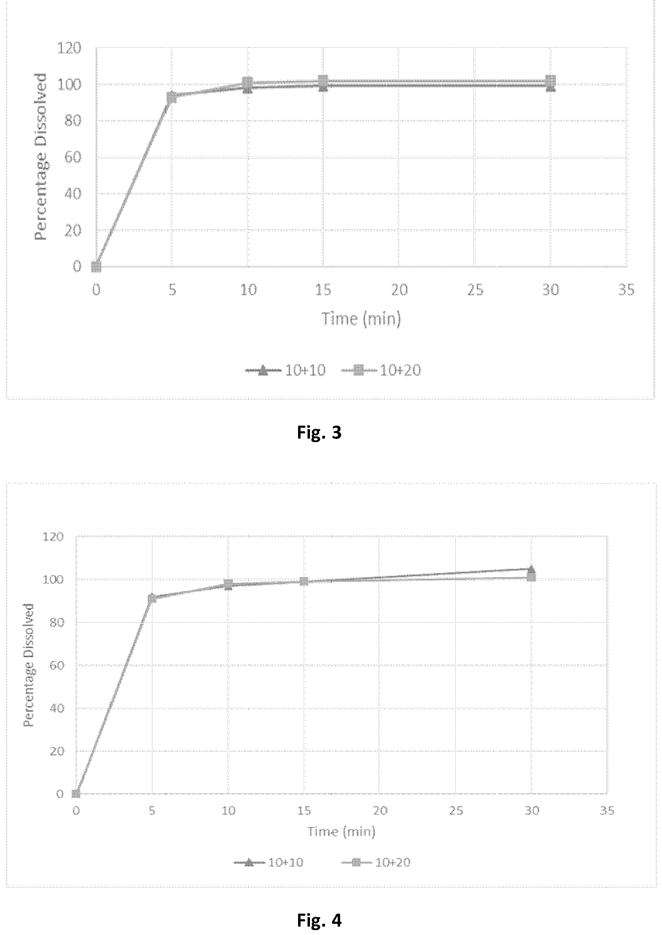

**Figure 3** shows dissolution profiles of Donepezil hydrochloride released from formulations according to Example 1 of the present invention.
**Figure 4** shows dissolution profiles of Memantine hydrochloride released from formulations according to Example 1 of the present invention.

### DETAILED DESCRIPTION

The present disclosure relates to an immediate release fixed-dose combination pharmaceutical composition comprising the active pharmaceutical ingredients (APIs) Memantine and Donepezil, for the treatment of moderate to severe Alzheimer's disease.

In a preferred embodiment, Memantine and Donepezil are utilized in their hydrochloride salts form, i.e. as Memantine hydrochloride (Memantine HCl) and Donepezil hydrochloride (Donepezil HCl).

In an even more preferred embodiment, Memantine HCl is utilized in crystalline Form I according to the document WO 2006/076560 and Donepezil HCl is utilized in crystalline Form I according to the document US 6,140,321.

Memantine may be manufactured according to processes known in the art, e.g. as disclosed in the document EP 0 392 059. Memantine HCl in crystalline Form I may be manufactured according to the document WO 2006/076560.

Donepezil may be manufactured according to processes known in the art, e.g. as disclosed in the document EP 0 296 560. Donepezil HCl in crystalline Form I may be manufactured according to the document US 6,140,321.

In an embodiment, the immediate release oral fixed-dose combination pharmaceutical composition of Memantine and Donepezil according to the present disclosure exhibits a dissolution rate of preferably more than 50% (expressed as percentage of labeled claim of each drug) within 30 minutes, preferably more than 60% within 30 minutes, most preferably more than 70% within 30 minutes.

More preferably, the dissolution rate of the immediate release oral fixed-dose combination pharmaceutical composition of Memantine and Donepezil is more than 70% within 5 minutes, even more preferably more than 75% within 5 minutes, most preferably more than 80% within 5 minutes.

In one embodiment, the immediate release oral fixed-dose combination pharmaceutical composition of Memantine and Donepezil contains 4.56 mg to 9.12 mg of Donepezil. In other embodiment, Donepezil HCl is present in amounts ranging from 5 mg to 10 mg per unit (dose).

In another embodiment, the immediate release oral fixed-dose combination pharmaceutical composition of Memantine and Donepezil contains 4.16 mg to 16.62 mg of Memantine. In other embodiment, Memantine HCl is present in amounts ranging from 5 mg to 20 mg per unit (dose).

In one embodiment, the immediate release oral fixed-dose combination pharmaceutical composition according to the present disclosure does not contain lactose.

In one aspect of the present disclosure, the immediate release oral fixed-dose combination pharmaceutical composition has a release profile for Donepezil which gives rise to a 90% confidence interval (90% Cl) for the ratio of the geometric means of AUC (area under the curve of plasma concentration versus time) for test product (present disclosure) and Aricept^{®} (reference product), from 80.00 to 125.00%, more preferably from 90.00 to 111.00%.

In another aspect of the disclosure, the immediate release oral fixed-dose combination pharmaceutical composition has a release profile for Donepezil which gives rise to a 90% CI for the ratio of the geometric means of Cmax (maximum plasma concentration) for test product (present disclosure) and Aricept^{®} (reference product), from 80.00 to 125.00%, more preferably from 90.00 to111.00%.

In another aspect of the present disclosure, the immediate release oral fixed-dose combination pharmaceutical composition has a release profile for Memantine which gives rise to a 90% CI for the ratio of the geometric means of AUC (area under the curve of plasma concentration versus time) for test product (present disclosure) and Ebixa^{®}(reference product), from 80.00 to 125.00%, more preferably from 90.00 to 111.00%.

In one further aspect of the present disclosure, the immediate release oral fixed-dose combination pharmaceutical composition has a release profile for Memantine which gives rise to a 90% confidence interval (90% Cl) for the ratio of the geometric means of Cmax (maximum plasma concentration) for test product (present disclosure) and Ebixa^{®} (reference product), from 80.00 to 125.00%, more preferably from 90.00 to 111.00%.

In an embodiment, besides the combination of Donepezil and Memantine, the pharmaceutical compositions according to the present disclosure may further comprise one or more disintegrant(s), one or more binder(s), one or more diluent(s), one or more lubricant(s), one or more glidant(s) and optionally one or more other pharmaceutically acceptable excipient(s). In the context of the present invention, the terms active(s), disintegrant(s), binder(s), diluent(s), lubricant(s), glidant(s) etc. shall be understood as including a single compound but also multiple compounds.

In an embodiment, the pharmaceutically acceptable disintegrant(s) include, but are not limited to starch, carboxymethyl cellulose (carmellose), crosslinked carboxymethyl cellulose (croscarmellose), sodium starch glycolate, low substituted hydroxypropyl cellulose (L-HPC), carboxymethyl starch, polyvinylpyrrolidone, crospovidone, and mixtures thereof. Preferably, the pharmaceutical composition comprises from 2 wt. % to 30 wt. % of maize starch and/or croscarmellose sodium disintegrants. In particularly preferred embodiments, the pharmaceutical composition comprises from 5 wt. % to 25 wt. % of maize starch and from 5 wt. % to 20 wt. % of croscarmellose sodium.

In an embodiment, the pharmaceutically acceptable binder(s) include, but are not limited to hydroxypropyl cellulose (HPC), low-substituted hydroxypropyl cellulose (L-HPC), hydroxypropyl methylcellulose (HPMC), methyl cellulose, hydroxyethyl cellulose, ethyl cellulose, polyethylene glycol, maltodextrin, pregelatinized starch, polymethacrylates, sodium alginate, polyvinylpyrrolidone (povidone), vinylpyrrolidone/vinylacetate copolymer (copovidone) and mixtures thereof. The preferred binder is hydroxypropyl cellulose (HPC).

In a preferred embodiment, the amount of one or more binder(s) is from 0.5 wt. % to 10 wt. %, more preferably from 1 wt. % to 5 wt. %. In a particularly preferred embodiment, the pharmaceutical composition of the present disclosure comprises from 1 wt. % to 4 wt. % of hydroxypropyl cellulose (HPC).

In an embodiment, the pharmaceutically acceptable diluent(s) include, but are not limited to, microcrystalline cellulose (MCC), silicified microcrystalline cellulose (SMCC), mannitol, sorbitol, lactitol, xylitol, isomalt, dextrin, and mixtures thereof. Preferably, the pharmaceutical composition of the present disclosure comprises from 5.0 wt. % to 80.0 wt. % of one or more diluents.

In an embodiment, the pharmaceutical composition of the present disclosure comprises from 5 wt. % to 20 wt. % of microcrystalline cellulose relative to the total weight of the composition. In an embodiment, the pharmaceutical composition of the present disclosure comprises from 45.0 wt. % to 70 wt. % of mannitol, preferably from 45.0 wt. % to 60 wt. % of mannitol, more preferably from 45.0 wt. % to 55 wt. % of mannitol relative to the total weight of the composition.

In an embodiment, the preferred diluents are mannitol, microcrystalline cellulose (MCC) and mixtures thereof. In the pharmaceutical compositions of the present disclosure, the sum of the amount of the pharmaceutically active ingredients and the amounts of diluents (cellulose microcrystalline and mannitol) is from 190 mg to 210 mg. Furthermore, the sum of the amount of Memantine or a pharmaceutically acceptable salt thereof and the amount of diluents (cellulose microcrystalline and mannitol) is from 180 mg to 200 mg.

In an embodiment, the suitable lubricant(s) include, but are not limited to, magnesium stearate, calcium stearate, sodium stearate, stearic acid, sodium glyceryl behenate, sodium stearyl fumarate and mixtures thereof. Preferably, the pharmaceutical composition of the present disclosure comprises from 0.1 wt. % to 5 wt. % of one or more lubricant(s). In a particularly preferred embodiment, the pharmaceutical composition of the present disclosure comprises from 0.1 wt. % to 5 wt. % of magnesium stearate relative to the total weight of the composition.

In an embodiment, the pharmaceutical composition of the present disclosure optionally comprises pharmaceutically acceptable glidant(s) such as colloidal silicon dioxide, talc, magnesium carbonate and mixtures thereof. The amount of glidant(s) is preferably from 0.1 wt. % to 1.5 wt. %, relative to the total weight of the composition.

Another aspect of the present disclosure relates to processes for manufacturing the immediate release oral fixed-dose pharmaceutical composition.

In one embodiment, the process of preparing the pharmaceutical composition of the present disclosure comprises a) mixing pharmaceutically effective amounts of Donepezil and Memantine, or a pharmaceutically acceptable salt thereof, with at least one diluent, optionally one or more binders, and optionally one or more disintegrants; b) adding a lubricant, and optionally one or more pharmaceutically acceptable excipients, and mixing the compositions together; c) compressing the final mixture into a plurality of tablets; d) optionally coating the tablets.

In another embodiment, the process of preparing the pharmaceutical composition of the present disclosure comprises a) mixing pharmaceutically effective amounts of Donepezil and Memantine, or a pharmaceutically acceptable salt thereof, with at least one diluent, optionally one or more binders, and, optionally, one or more disintegrants; b) granulating the previous mixture, in manner known to the one known in the art, to obtain a plurality of granules; c) mixing the granules with a lubricant, and optionally with one or more pharmaceutically acceptable excipients; d) compressing the final mixture into a plurality of tablets; e) optionally coating the tablets.

In an embodiment, the mixing step before granulation is performed in a diffusion mixer, in a high shear granulator or in a fluidized bed dryer, preferably in a high shear mixer in a manner known in the art. When wet granulation is used to prepare the granules, the obtained granules are dried before the subsequent steps. Optionally, the wet granules may require an intermediate wet milling step, before drying, through a relatively coarse mesh to break-up oversized agglomerates and promote a faster drying process. The granules may be dried, for example, in a fluidized bed dryer or in a static bed oven. Preferably, the granules are dried in a fluidized bed dryer. Optionally, the dried granules are milled in a suitable mill. Preferable mills are impact mills, cutting mills or sieving mills. Most preferably, a sieving mill is used. The tablets are preferably coated in manner known to the skilled person. Preferably, the coating contains color pigments.

### EXAMPLES

EXAMPLE 1 - Qualitative and Quantitative Composition of the lactose-free fixed-dose combination compositions according to the present disclosure

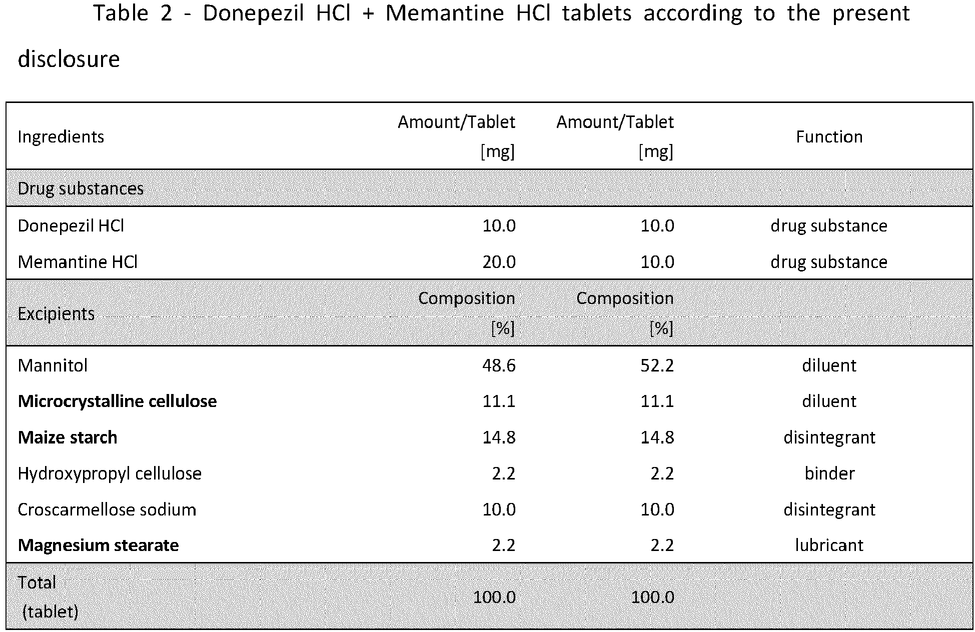

The pharmaceutical compositions of example 1 were prepared by a conventional wet-granulation technique comprising the following steps: a) mixing Donepezil HCl and Memantine HCl, mannitol, microcrystalline cellulose, maize starch, hydroxypropyl cellulose and croscarmellose; b) granulating the mixture with water to obtain a wet mass; c) optionally milling the wet mass c) drying the wet mass; e) milling the dried granules; f) blending the dried granules with lubricant; f) compressing the lubricated mixture into a plurality of tablets; g) optionally, coating the tablets by processes well known in the art.

### REFERENCE EXAMPLE (R1)

Lactose containing formulations were manufactured according to the steps described in Example 1. In example R1, mannitol was replaced with lactose.

The obtained tablets were incubated in oven at 60 °C. After a preset time, the samples were tested for Memantine lactose-adduct (MLA). The results (%) are summarized in the table below.

The sum of the amount of pharmaceutically active ingredients and the amounts of diluents (cellulose microcrystalline and lactose monohydrate) is from 190 mg to 210 mg. Furthermore, the sum of the amount of Memantine or a pharmaceutically acceptable salt thereof, and the amount of diluents (cellulose microcrystalline and lactose monohydrate) is from 180 mg to 200 mg.

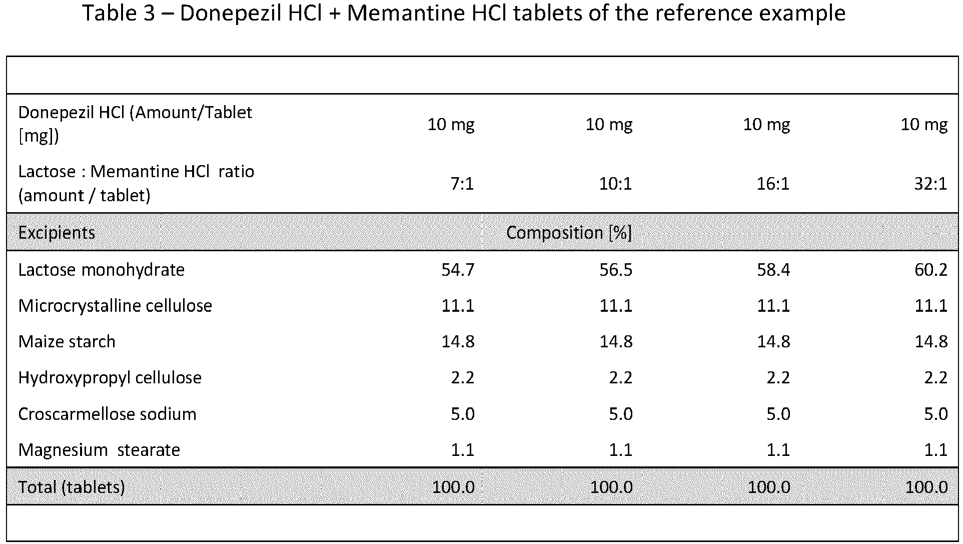

**Table 4 - Stability of Lactose containing formulations**

| **Ratio Diluent: Memantine** | | **60 °C/100%RH (4 days)** |
|---|---|---|
| **7:1** (13% Lactose) | Granules | 34.4 |
| | Tablets | 35.2 |
| **29:1** (54% Lactose) | Granules | 24.9 |
| | Tablets | 28.6 |
| **32:1** (60% Lactose) Memantine added into the external phase | Granules | 22.7 |
| | Tablets | 26.7 |

**Table 5 - Stability of Lactose containing formulations**

| Ratio Diluent: Memantine | | 60 **°C** /100%RH (overnight) |
|---|---|---|
| 7:1 (13% Lactose) | Tablets | 3.6 |
| 29:1 (54% Lactose) | Tablets | 1.7 |

Noticeably, these experiments show that the lower the lactose quantity in the fixed-dose composition, the higher the quantity of the impurity Memantine-lactose adduct (MLA).

When lactose was replaced by mannitol, surprisingly no impurity Memantine-lactose adduct (MLA) was detected (Table 6). As such, the composition of the present disclosure is more stable.

**Table 6 - Content of Memantine-lactose adduct (MLA)**

| Ratio Diluent: Memantine | | **60 °C/100%RH (4 days)** |
|---|---|---|
| **32:1** (60% Lactose) Memantine added into the external phase | Granules | 22.7 |
| | Tablets | 26.7 |
| **32:1** (60% Mannitol)(*) | Granules | N.D |
| | Tablets | N.D |

| | | |
|---|---|---|
| (N.D.=not detected) (^{∗}) composition prepared according to example 1. | | |

### Dissolution testing

The in-vitro dissolution of tablets of EXAMPLE 1 containing Donepezil HCl and Memantine HCl was determined according the method described in table 7.

**Table 7 - Parameters of the in-vitro dissolution of tablets**

| **Reference** | **Apparatus** | **Speed [RPM]** | **Medium / pH-value** | **Volume [ml]** | **Sampling Times [min]** |
|---|---|---|---|---|---|
| USP | II (Paddle) | 50 | 0.1 N HCl / pH 1 | 1000 | 5, 10, 20, 30 |

**Table 8 - Memantine dissolution data (%) of tablets of the example 1**

| **Active** | **Memantine, pH 1** | |
|---|---|---|
| **Time** | **10 mg + 10 mg** | **10 mg + 20 mg** |
| 0 | 0 | 0 |
| 5 | 92 | 91 |
| 10 | 97 | 98 |
| 15 | 99 | 99 |
| 30 | 105 | 101 |

**Table 9 - Donepezil dissolution data (%) of tablets of the example 1**

| **Active** | **Donepezil, pH 1** | |
|---|---|---|
| **Time** | **10 mg + 10 mg** | **10 mg + 20 mg** |
| 0 | 0 | 0 |
| 5 | 94 | 93 |
| 10 | 98 | 101 |
| 15 | 99 | 102 |
| 30 | 99 | 101 |

**Table 10 - Memantine and Donepezil dissolution data (%) of tablets of the reference example (Lactose: Memantine HCl ratio=7.38)**

| **Active** | **Memantine pH 1** | **Donepezil pH 1** |
|---|---|---|
| **Time** | **10 mg + 20 mg** | **10 mg + 20 mg** |
| 0 | 0 | 0 |
| 5 | 63 | 67 |
| 10 | 89 | 93 |
| 15 | 95 | 99 |
| 30 | 100 | 103 |

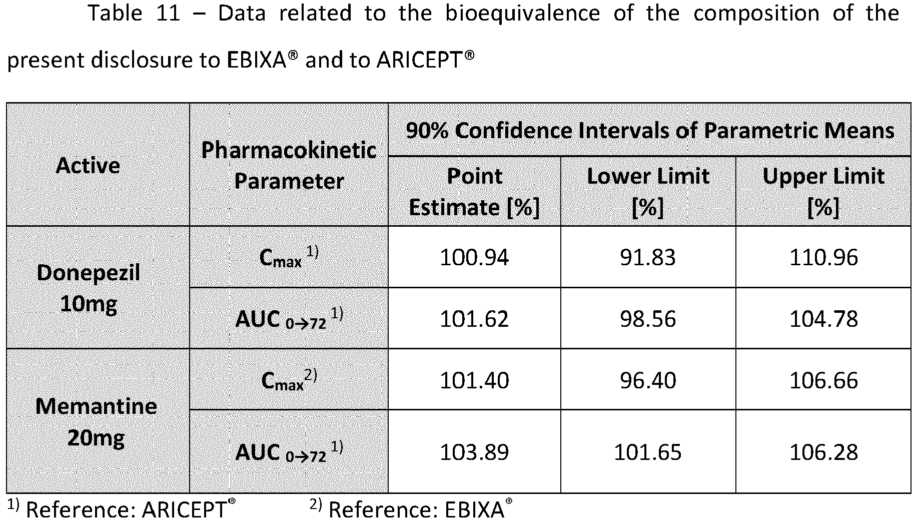

It is well known in the scientific community that Mannitol may render APIs adsorption difficult, and it is in fact described in the Guideline for the Investigation of Bioequivalence (CPMP/EWP/QWP/1401/98 Rev. 1/ Corr) that Mannitol may affect gastrointestinal transit and hence the bioavailability of a drug.

**Table 12 - Data related to the dissolution and stability of the composition**

| | % dissolved after 5 minutes | | At release | | 40 °C /75°/ RH - 6months | |
|---|---|---|---|---|---|---|
| % (W/W) Manitol | Donepezil | Memantine | Donepezil Total Impurities (%) (NMT 3.0%) | Memantine Total Impurities (%) (NMT 2.0%) | Donepezil Total Impurities (%) (NMT 3.0%) | Memantine Total Impurities (%) (NMT 2.0%) |
| 54.1 | 97 | 99 | <0.1 | 0.05 | 0.19 | <0.05 |
| 52.2 | 94 | 91 | <0.1 | <0.05 | <0.1 | <0.05 |
| 50.4 | 91 | 93 | <0.1 | <0.05 | 0.12 | <0.05 |
| 48.5 | 91 | 92 | <0.1 | <0.05 | 0.13 | <0.05 |

Surprisingly, in the presence of Mannitol, Donepezil and Memantine were found to be bioequivalent to the mono-products, thus allowing the conclusion that Mannitol does not affect the bioavailability of both substances when formulated as an immediate release fixed-dose combination composition. The composition according to the present disclosure does not produce different absorption levels as compared to the products containing only Memantine or only Donepezil.

The results show that surprisingly the composition of the present disclosure allows an improved dissolution rate, an improved disintegration rate, and an improved shelf storage stability.

Accordingly, preferred embodiments of the present disclosure refer to immediate release oral pharmaceutical compositions that are bioequivalent as compared to only Memantine (namely, to EBIXA^{®} reference product) and as compared to only Donepezil (namely, to ARICEPT^{®} reference product).

The term "comprising" whenever used in this document is intended to indicate the presence of stated features, integers, steps, components, but not to preclude the presence or addition of one or more other features, integers, steps, components or groups thereof.

Where ranges are given, endpoints are included. Furthermore, it is to be understood that unless otherwise indicated or otherwise evident from the context and/or the understanding of one of ordinary skill in the art, values that are expressed as ranges can assume any specific value within the stated ranges in different embodiments of the invention, to the tenth of the unit of the lower limit of the range, unless the context clearly dictates otherwise. It is also to be understood that unless otherwise indicated or otherwise evident from the context and/or the understanding of one of ordinary skill in the art, values expressed as ranges can assume any subrange within the given range, wherein the endpoints of the subrange are expressed to the same degree of accuracy as the tenth of the unit of the lower limit of the range.

The disclosure should not be seen in any way restricted to the embodiments described and a person with ordinary skill in the art will foresee many possibilities to modifications thereof.

The above described embodiments are combinable.

The following claims further set out particular embodiments of the disclosure.

## Claims

1. An immediate release oral fixed-dose combination pharmaceutical composition, for use in treatment or therapy of moderate to severe Alzheimer's disease, comprising:
from 4.16 mg to 20 mg of Memantine or pharmaceutically acceptable salt; and
from 4.56 mg to 10 mg of Donepezil or pharmaceutically acceptable salt;
wherein Memantine or pharmaceutically acceptable salt and Donepezil or pharmaceutically acceptable salt are the sole active substances;
wherein the pharmaceutical composition is lactose-free;
and less than 1.0 wt.% of reducing sugars relative to the total weight of the composition;
wherein the dissolution rate of Memantine and Donepezil in pH 1 dissolution test solution is more than 90% within 5 minutes.

2. The composition according to the previous claim wherein the dissolution rate of Memantine and Donepezil is more than 91% within 5 minutes.

3. The composition according to any the previous claims further comprising 45.0 wt. % to 70.0 wt. % of mannitol.

4. The composition according to any the previous claims further comprising 45.0 wt. % to 60.0 wt. % of mannitol, preferably from 45.0 wt. % to 55.0 wt. % of mannitol.

5. The composition according to the previous claims comprising less than 0.6 wt.% of reducing sugars relative to the total weight of the composition, preferably less than 0.4 wt.%.

6. The composition according to the previous claims comprising less than 0.3 wt.% of reducing sugars.

7. The composition according to any of the previous claims, wherein the bioequivalence of Memantine in the composition is equivalent to EBIXA^{®}, and the bioequivalence of Donepezil in the composition is equivalent to ARICEPT^{®}.

8. The composition according to any of the previous claims, wherein the pharmaceutically acceptable salt for Memantine is Memantine hydrochloride, preferably in amounts from 5 mg to 20 mg per dose.

9. The composition according to any of the previous claims, wherein the pharmaceutically acceptable salt for Donepezil is Donepezil hydrochloride, preferably in amounts from 5 mg to 10 mg per dose.

10. The composition according any of the previous claims, wherein the dissolution rate of Memantine and Donepezil in pH 1 dissolution test solution is more than 50% within 30 minutes, preferably more than 60% within 30 minutes, most preferably more than 70% within 30 minutes.

11. The composition according any of the previous claims, comprising less than 2.5 wt. % of Memantine-Lactose Adduct (MLA) relative to the weight of Memantine, preferably less than 1.4 wt. %, more preferably less than 1.0 wt. %.

12. The composition according to any of the previous claims further comprising:
one or more disintegrant(s);
one or more binder(s);
one or more diluents(s);
one or more lubricant(s);
optionally one or more glidant(s);
other pharmaceutically acceptable excipient(s).

13. The composition according to any of the claims 15, wherein:
one or more disintegrant(s) are selected from a list consisting of: starch, carboxymethyl cellulose (carmellose), crosslinked carboxymethyl cellulose (croscarmellose), sodium starch glycolate, low substituted hydroxypropyl cellulose (L-HPC), carboxymethyl starch, polyvinylpyrrolidone, crospovidone, and mixtures thereof;
one or more binders(s) are selected from a list consisting of: hydroxypropyl cellulose (HPC), low-substituted hydroxypropyl cellulose (L-HPC), hydroxypropyl methylcellulose (HPMC) , methyl cellulose, hydroxyethyl cellulose, ethyl cellulose, polyethylene glycol, maltodextrin, pregelatinized starch, polymethacrylates, sodium alginate, polyvinylpyrrolidone (povidone), vinylpyrrolidone/vinylacetate copolymer (copovidone), and mixtures thereof;
one or more diluent(s) are selected from a list consisting of: microcrystalline cellulose (MCC), silicified microcrystalline cellulose (SMCC), sorbitol, lactitol, xylitol, isomalt, dextrin, and mixtures thereof; preferably microcrystalline cellulose;
one or more lubricant(s) are selected from a list consisting of: magnesium stearate, calcium stearate, sodium stearate, stearic acid, sodium glyceryl behenate, sodium stearyl fumarate, and mixtures thereof.

14. The composition according to any of the previous claims, wherein the composition is obtainable by wet granulation.

15. A process for the manufacture of a pharmaceutical composition according to any of claims 1 to 14, comprising the steps of:
preparing a homogenous blend comprising
Donepezil or a pharmaceutically acceptable salt, in particular Donepezil hydrochloride,
Memantine or a pharmaceutically acceptable salt, in particular Memantine hydrochloride,
one or more disintegrant(s), one or more binder(s), and one or more diluents(s);
obtaining granules by means of wet granulation;
drying the wet granules and sieving;
blending the granules with one or more lubricant(s);
compressing the final blend into a tablet;
optionally film-coating the tablet.
